# EUROPEAN PATENT SPECIFICATION

(11) **EP 1 339 342 B1**
(45) Date of publication and mention of the grant of the patent: **09.01.2008**
(21) Application number: 01997438.5
(22) Date of filing: 20.11.2001
(51) Int. Cl.: A61B 19/02

(54) **CLEAR MEDICAL PACKAGING**
DURCHSICHTIGE MEDIZINISCHE PRODUKTVERPACKUNG
EMBALLAGE MEDICAL TRANSPARENT

(30) Priority: 20.11.2000 US 716619; 20.11.2000 US 716687
(43) Date of publication of application: 03.09.2003
(73) Proprietor: Becton Dickinson and Company, Franklin Lakes, New Jersey 07417 (US)
(72) Inventor: HEYMAN, Peter, W., Florham Park, NJ 07932 (US); KORISCH, Marina, S., Wayne, NJ 07470 (US); ALCHAS, Paul, G., Wayne, NJ 07470 (US)
(74) Representative: Shanks, Andrew
(86) International application number: PCT/US2001/043234
(87) International publication number: WO 2002/042164

(56) References cited:
- EP-A- 0 266 688
- WO-A-99/45984
- DE-A1- 2 952 733
- US-A- 4 402 407

## Description

### FIELD OF THE INVENTION

The present invention relates to medical device packages. More particularly, the present invention relates to medical device packages having a gas permeable lid and a clear plastic tub.

### BACKGROUND OF THE INVENTION

Medical devices, such as prefillable syringes and the like are frequently packaged in multi-unit packages that must be surface decontaminated prior to introduction into a controlled sterile environment where the devices are filled with medication or otherwise further manipulated prior to use, or used in a surgical procedure.

There are numerous methods used to sterilize the devices within the packages, such as gas sterilization utilizing ethylene oxide or steam sterilization. However, different methods, such as ultraviolet light sterilization, are often used for decontamination of the surfaces of the packages prior to subsequent use. It would be desirable to have a medical device package capable of use in a multitude of sterilization and decontamination techniques and yield a high level of kill of microorganisms.

A common type of package utilized in the field of medical packaging is the peel-open package. Such a package commonly comprises a thermoplastic film that is formed to a desired package shape and a lid material is then sealed to the plastic film to contain the packaged product.

The common thermoplastic film packages described above are often utilized to package individual medical devices, as the film does not provide sufficient protective characteristics for use in a multiunit package which may house delicate items. Therefore, there is a need for a multiunit package, such as a tub, that can withstand the sterilization and decontamination processes and provide a sufficient mechanical barrier to prevent damage to the items held within the package.

The lid material used is commonly a non-woven fiber arranged such that it has a microporous structure, such as Tyvek®. The microporous fiber arrangement allows gas to penetrate but has a sufficiently small pore size to block the transfer of microorganisms.

The microporous fiber arranged lid is, therefore, useful in sterilization techniques utilizing a gas, such as the aforementioned ethylene oxide and steam sterilization techniques.

The Tyvek® lid, however, does not allow the transmission of ultraviolet light that may be used as a package surface decontamination technique. Specifically, the heat seal between the lid and tub cannot be made flush with the edge of the tub flange due to processing limitations and the potential for Tyvek® tearing during peel open if an unsealed lip of Tyvek® is not available for initiation of the peeling process. Therefore, there is a region outside of the heat seal, covered by Tyvek®, which is not maintained sterile with the contents of the package. This area can be inadvertently contaminated during manipulation of the package, as can the entire external surface of the package. However, unlike the remainder of the external surface, which can be directly exposed to ultraviolet light for decontamination, the area outside of the heat seal is blocked to the ultraviolet light by the opaque Tyvek® material on top and opaque tub material below.

Therefore, it would be desirable to utilize an ultraviolet-light transmissive plastic tub that can allow transmission of light to the underside of the heat seal area between the lid and tub to kill all microorganisms residing therein. Plastics which transmit light in the ultraviolet range typically transmit an even greater percentage of incident light in the visible (400-700 nanometer) range, resulting in a clear appearance.

An advantage of utilizing a clear plastic tub, as opposed to an opaque one is that it would allow for visual inspection of the contents of the package to determine if any of the items are damaged. This aspect offers quality control advantages to a manufacturer, as well as allow end users to ensure they are not receiving or using damaged items. Another advantage of utilizing a clear plastic tub would be the possible use of an automated visual quality system. For example, a package and its contents may be imaged and compared to a stored visual image to determine if the contents of the package are damaged. Such an automated system could provide a cost savings, as well as an increased quality control efficiency.

Alternatively, a Tyvek® lid having an ultraviolet-light transmitting plastic border that is heat sealed to a plastic tub would allow for light sterilization of the area between the lid and tub. Such a lid may be utilized with either a clear plastic tub, or with an opaque tub and allow for light to penetrate the area around the heat seal.

Another problem associated with Tyvek® lids that are known in the art, is the possibility of the Tyvelc® lid shearing or tearing when peeled to open the package and thereby generating unacceptable particulate matter that may contaminate the contents of the package.

It is known in the art to coat a Tyvek® lid in an effort to reduce particulate generation, as well as prevent the lid from separating or delaminating when the heat seal strength between the lid and tub is too great. A disadvantage to this coating technique is that it has the tendency to reduce the pore size of the Tyvek® material; thereby preventing the necessary transmission of gas across the lid material or increasing the cycle time of a sterilization procedure.

It would be desirable to have a gas permeable uncoated Tyvek® lid that has a smooth continuous plastic border that is resistant to shearing and does not generate particulate matter when peeled away to access the contents.

A lid with a smooth continuous plastic border would also be desirable for use with an automated lid removal system, such as a hot knife or laser cutter that would cut the plastic border inside of the heat seal region, as an alternative to a peel-open technique.

US 4,402,407 relates to a sterilisation chest for surgeons instruments and the like. The container includes a generally open-top box having a peripheral flange about its top edge and an open channel extending along at least two of the opposite sides of the box in the peripheral flange.

WO 99/45984 relates to a method for manufacturing, filling and packaging medical containers. The medical devices are manufactured and annealed by heating in an oven which produces a clean device having a low bio-burden. Plastic medical devices and medical containers are formed by plastic moulding devices which produce a clean device.

DE 2952733 relates to sterilisable packaging made of a thermoplastic film for the medical or sterilising industry. The packaging consists of a deep-drawn beaker-shaped part with a flange-like outwardly protruding edge and a cover film which can be joined to this edge by hot sealing or welding.

EP 0266688 relates to a container for medical material. The container comprises a cover film of film-like material which hermitically seals an opening of the container and can be removed from the container. The cover has an opening with a porous membrane which can be welded to the material of the cover.

### SUMMARY OF THE INVENTION

According to a first aspect of the present invention there is provided a medical device package comprising a lid having a first gas permeable central portion and an ultraviolet transparent plastic film attached to the central portion, each of said first gas permeable portion and said plastic film permitting one of a disinfectant gas and ultraviolet light to pass through said lid to sterilize an area beneath said lid.

It is a further object of the present invention to provide a medical device package having a microporous, gas permeable lid with an ultraviolet-transmissive plastic border to facilitate the transmission of light during a decontamination process.

The present invention meets these objects by providing a medical device package having the features of claim 1.

The present invention therefore includes a lid having a gas permeable central portion and a transparent plastic film attached to the central portion along its periphery. The plastic film overlaps the central portion when attached to assure a reliable bond of the two items. The transparent plastic film has a high shear resistance to avoid producing particulate matter when an opening force is applied. The transparent plastic film also allows transmission of ultraviolet light. These and other features of the present invention can be best understood from the following specification and drawings.

### BRIEF DESCRIPTION OF THE DRAWINGS

Figure 1 is a perspective view of the medical device package of the present invention.
Figure 2 is a sectional view of the medical device package of the present invention.

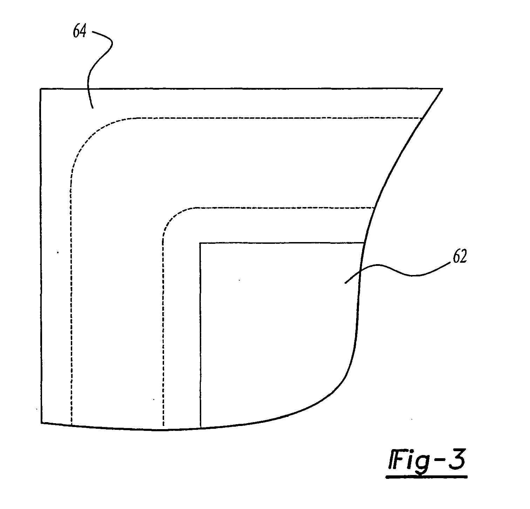

Figure 3 is a plan view of a corner of the medical device package of the present invention.
Figure 4 is perspective view of the tub of the present invention.
Figure 5 is a plan view of the tub of the present invention showing the contours that facilitate release of the tub from a mold.

### DETAILED DESCRIPTION OF THE PREFERRED EMBODIMENT

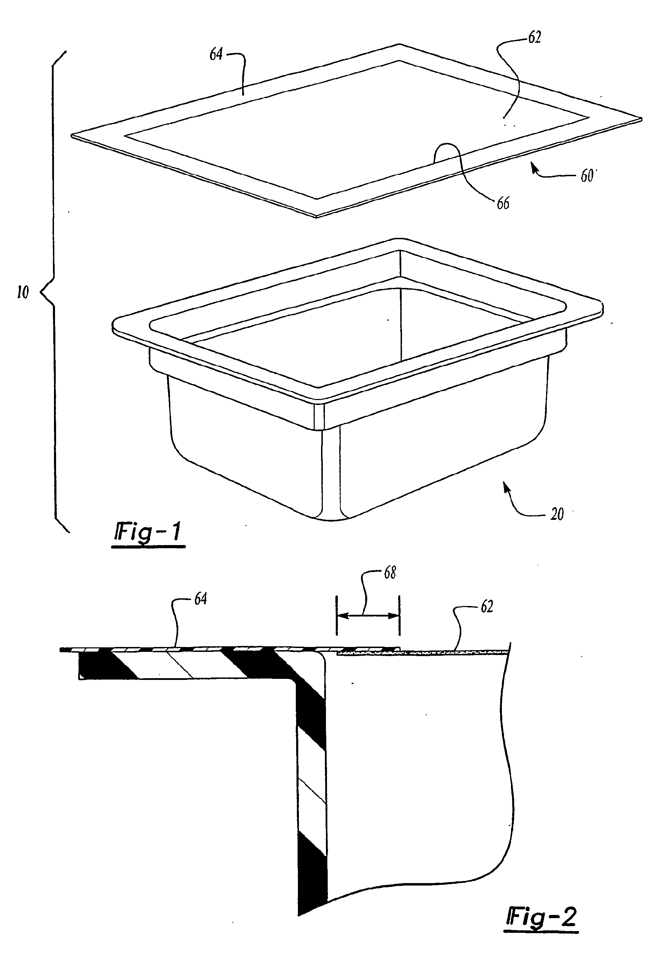

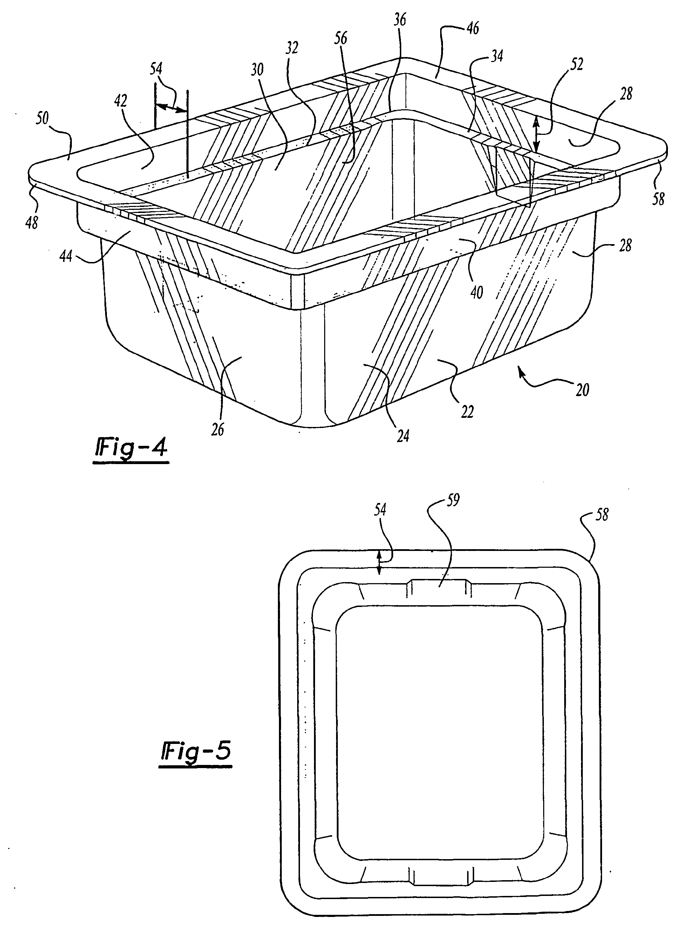

There is shown generally in Figure 1 at 10 the medical device package of the present invention. The medical device package 10 includes a tub 20 and a lid 60. With reference to Figures 1 and 4, the tub 20 has a planar base 22 from which four walls 24, 26, 28, 30 extend upward at approximately ninety degrees. The four walls 24, 26, 28, 30 terminate at an upper end 32 to form a first outwardly extending flange 34. The first flange intersects the upper end 32 of the walls at approximately ninety degrees and extends outwardly therefrom. There are four top walls 38, 40, 42, 44 extending upwardly at approximately ninety degrees from the outer edge 36 of the first outwardly extending flange 34. The four top walls 38, 40, 42, and 44 terminate at a second upper end 46 to form a second outwardly extending flange 48. The second outwardly extending flange 48 intersects the second upper end 46 at approximately ninety degrees and extends outwardly therefrom. The second outwardly extending flange has an upper surface 50 for mounting or adhering a lid 60 to the tub 20.

The gap 52 defined by the vertical distance between the first flange 34 and second flange 48 provides an area for use of an interior holding apparatus. Such an apparatus could hold the contents, for example syringes, and maintain the spatial arrangement of the contents of the package. The gap 52 also provides a depth for an automated opening tool to be utilized without contacting and possibly damaging the contents of the tub 20.

The horizontal or outward distance between the first flange 34 and second flange 48 defines a second gap 54. The contents of the tub 20 are contained inside the space 56 defined by the four walls 24, 26, 28, 30 and are inboard of the first flange 34. The gap 54, similar to the gap 52 defines a buffer zone when utilizing an automated opening tool such as a hot knife, or laser cutter to prevent damage of the contents of the tub 20.

The tub 20 is preferably formed of a plastic resin that allows the transmission of both visible and ultraviolet light. Examples of suitable materials for visible light transmission only, include high melt flow polycarbonate and high melt flow copolyester, which also exhibit good impact strength and overall durability. A particularly preferred resin of the present invention is methylpentene copolymer which transmits both visible and ultraviolet light and provides good mechanical protection of the contents of the tub. Specifically, the methylpentene copolymer demonstrates good transmissive properties at the microorganism killing ultraviolet wavelengths at 254 nanometers.

The tub 20 is preferably formed utilizing an injection molding process or a reaction injection molding process. In an injection molding process, the resin is injected into a mold under a specified temperature and pressure and allowed to cure to form a solid article.

The tub 20 of the present invention, preferably, has radiused corners 58 both on its exterior and interior to facilitate removal of the tub 20 from the mold during manufacture. The radiused corners 58 also prevent the formation of sharp edges that may cause damage to the contents of the tub 20, as well as, pose a threat of possible injury to a person handling the tub 20. The tub 20 may also include contoured portions 59, as shown in Figures 4 and 5, to facilitate removal of the tub 20 from the mold during manufacture.

The lid 60 of the present invention comprises a gas permeable microfiber central portion 62 and a transparent plastic film 64 attached to the central portion 62 along its periphery 66.

The central portion 62 preferably comprises Tyvek®, a polyolefin microfiber material produced by E.I. Dupont de Nemours and Company of Wilmington, Delaware. Even more preferably, the central portion 62 comprises uncoated Tyvek® 1073B.

The transparent plastic film 64 is attached along the periphery 66 of the central portion using adhesives, glues or other bonding agents or may be heat welded. The transparent plastic film 64 should overlap 68 the central portion at least five millimeters to assure an adequate bonding between the two items.

The width of the transparent film 64 is variable, dependent upon the application to which it is to be associated. For example, if the lid 60 is to be removed manually utilizing a peel-away procedure, a narrow width in the range of 10-20 millimeters may be utilized. The 10-20 millimeter width would allow sufficient light to enter to sterilize the area just outside of the heat seal, but not be excessively wide from an economic perspective. If the lid 60 is to be utilized in an alternate application such as an automated opening operation, a wider width may be utilized to prevent possible shearing of the central portion 62, thereby producing particulate matter. A suitable width for the transparent film 64 is in the range of 20 to 30 millimeters for an automated procedure.

The film 64 should allow transmission of light in the ultraviolet spectrum for surface decontamination procedures. The film 64 should also have a sufficient shear strength to resist accidental tearing, but be capable of easy opening utilizing an automated system.

The present invention has been described in accordance with the relevant legal standards, thus the foregoing description is exemplary rather than limiting in nature. Variations and modifications to the disclosed embodiment may become apparent to those skilled in the art. Accordingly, the scope of legal protection afforded this invention can only be determined by studying the following claims.

## Claims

1. A medical device package (10) comprising a lid (60) having a first gas permeable central portion (62) and an ultraviolet transparent plastic film (64) attached to the central portion (62), each of said first gas permeable portion (62) and said plastic film (64) permitting one of a disinfectant gas and ultraviolet light to pass through said lid (60) to sterilize an area beneath said lid (60).

2. A medical device package (10) as recited by claim 1, further comprising a tub (20) having a base (22), side walls (24,26,28,30) intersecting the base (22) and terminating at a first flange (34), top walls (38,40,42,44) extending from the first flange (34), said top walls (38,40,42,44) terminating at a second flange (48), said lid (60) being in sealing engagement with said second flange (48).

3. A medical device package (10) as recited by claim 1, wherein said transparent plastic film (64) is overlappingly attached along a periphery of said first portion.

4. A medical device package (10) as recited by claim 1, wherein said transparent film (64) has a high shear resistance to avoid producing particulate matter when a force is applied to said transparent film (64).

5. A medical device package (10) as recited by claim 2, wherein said tub (20) is a plastic selected from one of high melt flow polycarbonate, high melt flow copolyester, methylpentene copolymer, and thermoplastic.

6. A medical device package (10) as recited by claim 2, wherein said tub (20) is a plastic that permits transmission of light having a wavelength ranging from 254 to 700 nanometers.

7. A medical device package (10) as recited by claim 2, wherein said tub (20) is a plastic capable of withstanding exposure to one of steam and ethylene oxide sterilization without degradation of the plastic.

8. A medical device package (10) as recited by claim 1, wherein said first gas permeable portion is Tyvek®.

9. A medical device package (10) as recited by claim 1, further comprising:
a tub (20) having a four-side base (22); and
four walls (24,26,28,30) intersecting the base (22) at approximately ninety degrees and extending therefrom and terminating at a first outwardly extending flange (34) which extends from the four walls (24,26,28,30) at approximately ninety degrees; the first outwardly extending flange (34) terminating at four top walls (38,40,42,44) that extend from the first outwardly extending flange (34) at approximately ninety degrees and terminating at a second outwardly extending flange (34) that extends from the four top walls (38,40,42,44) at approximately ninety degrees; and
said tub (20) being formed of a clear plastic that is capable of withstanding exposure to steam and ethylene oxide sterilization environments without degradation of the tub (20).

10. The medical device package (10) of claim 9, wherein the tub (20) comprises a plastic which transmits visible light at from 100 to 700 nanometers.

11. A medical device package (10) as recited by claim 1, further comprising:
a tub (20) having a four-side base (22); and
four walls (24,26,28,30) intersecting the base (22) at approximately ninety degrees and extending therefrom and terminating at a first outwardly extending flange (34) which extends from the four walls at approximately ninety degrees; the first outwardly extending flange (34) terminating at four top walls (24,26,28,30) that extend from the first outwardly extending flange (34) at approximately ninety degrees and terminating at a second outwardly extending flange (48) that extends from the four top walls (24,26,28,30) at approximately ninety degrees; and
radiused intersections and corners for removing sharp corners and facilitating removal of the tub (20) from a mold; and
said tub (20) being formed of a clear, ultraviolet light transmitting plastic that is capable of withstanding exposure to steam and ethylene oxide sterilization environments without degradation of the tub (20).

## Patentansprüche

1. Medizinische Geräteverpackung (10), die einen Deckel (60) umfasst, der einen ersten, gasdurchlässigen, Mittelabschnitt (62) und eine an dem Mittelabschnitt (62) befestigte, ultraviolett durchscheinende, Kunststoff-Folie (64) hat, wobei sowohl der erste, gasdurchlässige, Abschnitt (62) als auch die Kunststoff-Folie (64) ermöglichen, dass entweder ein desinfizierendes Gas oder ultraviolettes Licht durch den Deckel (60) hindurchgeht, um einen Bereich unter dem Deckel (60) zu desinfizieren.

2. Medizinische Geräteverpackung (10) nach Anspruch 1, die ferner eine Wanne (20) umfasst, die eine Basis (22), Seitenwände (24, 26, 28, 30), welche die Basis (22) überschneiden und an einem ersten Flansch (34) enden, obere Wände (38, 40, 42, 44), die sich von dem ersten Flansch (34) aus erstrecken, hat, wobei die oberen Wände (38, 40, 42, 44) an einem zweiten Flansch (48) enden, wobei der Deckel (60) in Dichtungseingriff mit dem zweiten Flansch (48) ist.

3. Medizinische Geräteverpackung (10) nach Anspruch 1, wobei die durchscheinende Kunststoff-Folie (64) überlappend längs eines Umfangs des ersten Abschnitts befestigt ist.

4. Medizinische Geräteverpackung (10) nach Anspruch 1, wobei die durchscheinende Folie (64) eine höhere Scherfestigkeit hat, um ein Erzeugen von Feststoffen zu vermeiden, wenn auf die durchscheinende Folie (64) eine Kraft ausgeübt wird.

5. Medizinische Geräteverpackung (10) nach Anspruch 2, wobei die Wanne (20) ein Kunststoff ist, ausgewählt aus entweder Polycarbonat mit hohem Schmelzindex, Copolyester mit hohem Schmelzindex, Methylpenten-Copolymer oder thermoplastischem Kunststoff.

6. Medizinische Geräteverpackung (10) nach Anspruch 2, wobei die Wanne (20) ein Kunststoff ist, der ein Durchlassen von Licht ermöglicht, das eine Wellenlänge hat, die von 254 bis 700 Nanometer reicht.

7. Medizinische Geräteverpackung (10) nach Anspruch 2, wobei die Wanne (20) ein Kunststoff ist, der in der Lage ist, ohne Zersetzung des Kunststoffs einer Behandlung entweder mit Dampf- oder mit Ethylenoxid-Sterilisation standzuhalten.

8. Medizinische Geräteverpackung (10) nach Anspruch 1, wobei der erste, gasdurchlässige Abschnitt aus Tyvek® ist.

9. Medizinische Geräteverpackung (10) nach Anspruch 1, die ferner Folgendes umfasst:
eine Wanne (20), die eine Vierseitenbasis (22) hat, und
vier Wände (24, 26, 28, 30), welche die Basis (22) mit ungefähr neunzig Grad überschneiden und sich von derselben aus erstrecken und an einem ersten, sich nach außen erstreckenden, Flansch (34) enden, der sich von den vier Wänden (24, 26, 28, 30) aus mit ungefähr neunzig Grad erstreckt, wobei der erste, sich nach außen erstreckende, Flansch (34) an vier oberen Wänden (38, 40, 42, 44) endet, die sich von dem ersten, sich nach außen erstreckenden, Flansch (34) aus mit ungefähr neunzig Grad erstrecken und an einem zweiten, sich nach außen erstreckenden, Flansch (34) enden, der sich von den vier oberen Wänden (38, 40, 42, 44) aus mit ungefähr neunzig Grad erstreckt, und
wobei die Wanne (20) aus einem klaren Kunststoff geformt ist, der in der Lage ist, ohne Zersetzung der Wanne (20) einer Behandlung in Dampf- und Ethylenoxid-Sterilisationsumgebungen standzuhalten.

10. Medizinische Geräteverpackung (10) nach Anspruch 9, wobei die Wanne (20) einen Kunststoff umfasst, der sichtbares Licht bei 100 bis 700 Nanometer durchlässt.

11. Medizinische Geräteverpackung (10) nach Anspruch 1, die ferner Folgendes umfasst:
eine Wanne (20), die eine Vierseitenbasis (22) hat, und
vier Wände (24, 26, 28, 30), welche die Basis (22) mit ungefähr neunzig Grad überschneiden und sich von derselben aus erstrecken und an einem ersten, sich nach außen erstreckenden, Flansch (34) enden, der sich von den vier Wänden (24, 26, 28, 30) aus mit ungefähr neunzig Grad erstreckt, wobei der erste, sich nach außen erstreckende, Flansch (34) an vier oberen Wänden (24, 26, 28, 30) endet, die sich von dem ersten, sich nach außen erstreckenden, Flansch (34) aus mit ungefähr neunzig Grad erstrecken und an einem zweiten, sich nach außen erstreckenden, Flansch (48) enden, der sich von den vier oberen Wänden (24, 26, 28, 30) aus mit ungefähr neunzig Grad erstreckt, und
gerundete Überschneidungen und Ecken, um scharfe Ecken zu entfernen und das Entfernen der Wanne (20) aus einer Form zu erleichtern, und
wobei die Wanne (20) aus einem klaren, ultraviolettes Licht durchlassenden, Kunststoff geformt ist, der in der Lage ist, ohne Zersetzung der Wanne (20) einer Behandlung in Dampf- und Ethylenoxid-Sterilisationsumgebungen standzuhalten.

## Revendications

1. Emballage pour dispositif médical (10), comprenant un couvercle (60) comportant une première partie centrale perméable au gaz (62) et un film plastique transparent aux ultraviolets (64) fixé sur la partie centrale (62), chacune de ladite première partie perméable au gaz (62) et dudit film plastique (64) permettant le passage d'un gaz désinfectant et de la lumière ultraviolette à travers ledit couvercle (60) pour stériliser une zone située au-dessous dudit couvercle (60).

2. Emballage pour dispositif médical (10) selon la revendication 1, comprenant en outre un bac (20) comportant une base (22), des parois latérales (24, 26, 28, 30) coupant la base (22) et se terminant au niveau d'une première bride (34), des parois supérieures (38, 40, 42, 44) s'étendant à partir de la première bride (34), lesdites parois supérieures (38, 40, 42, 44) se terminant au niveau d'une deuxième bride (48), ledit couvercle (60) étant engagé de manière étanche dans ladite deuxième bride (48).

3. Emballage pour dispositif médical (10) selon la revendication 1, dans lequel ledit film plastique transparent (64) est fixé le long d'une périphérie de ladite première partie de sorte à chevaucher celle-ci.

4. Emballage pour dispositif médical (10) selon la revendication 1, dans lequel ledit film transparent (64) présente une résistance au cisaillement élevée pour empêcher la production de matières particulaires lors de l'application d'une force audit film transparent (64).

5. Emballage pour dispositif médical (10) selon la revendication 2, dans lequel ledit bac (20) est un plastique sélectionné dans le groupe constitué d'un polycarbonate à indice de fluidité élevé, d'un copolyester à indice de fluidité élevé, d'un copolymère de méthylpentène et un thermoplastique.

6. Emballage pour dispositif médical (10) selon la revendication 2, dans lequel ledit bac (20) est un plastique permettant la transmission de la lumière ayant une longueur d'onde comprise entre 254 et 700 nanomètres.

7. Emballage pour dispositif médical (10) selon la revendication 2, dans lequel ledit bac (20) est un plastique capable de résister à l'exposition à une stérilisation à la vapeur et à l'oxyde d'éthylène sans entraîner une dégradation du plastique.

8. Emballage pour dispositif médical (10) selon la revendication 1, dans lequel ladite première partie perméable au gaz est composée de Tyvek®.

9. Emballage pour dispositif médical (10) selon la revendication 1, comprenant en outre :
un bac (20) comportant une base à quatre côtés (22) ; et
quatre parois (24, 26, 28, 30) coupant la base (22) à environ quatre-vingt-dix degrés, s'étendant à partir de celle-ci et se terminant au niveau d'une première bride s'étendant vers l'extérieur (34), s'étendant à partir des quatre parois (24, 26, 28, 30) à environ quatre-vingt-dix degrés ; la première bride s'étendant vers l'extérieur (34) se terminant au niveau de quatre parois supérieures (38, 40, 42, 44) s'étendant à partir de la première bride s'étendant vers l'extérieur (34) à environ quatre-vingt-dix degrés et se terminant au niveau d'une deuxième bride s'étendant vers l'extérieur (34), s'étendant à partir des quatre parois supérieures (38, 40, 42, 44) à environ quatre-vingt-dix degrés ; et
ledit bac (20) étant composé d'un plastique transparent, capable de résister à l'exposition à des environnements de stérilisation à la vapeur et à l'oxyde d'éthylène sans entraîner une dégradation du bac (20).

10. Emballage pour dispositif médical (10) selon la revendication 9, dans lequel le bac (20) comprend un plastique transmettant la lumière visible ayant une longueur d'onde comprise entre 100 et 700 nanomètres.

11. Emballage pour dispositif médical (10) selon la revendication 1, comprenant en outre :
un bac (20) comportant une base à quatre côtés (22) ; et
quatre parois (24, 26, 28, 30) coupant la base (22) à environ quatre-vingt-dix degrés, s'étendant à partir de celle-ci et se terminant au niveau d'une première bride s'étendant vers l'extérieur (34), s'étendant à partir des quatre parois à environ quatre-vingt-dix degrés ; la première bride s'étendant vers l'extérieur (34) se terminant au niveau de quatre parois supérieures (24, 26, 28, 30) s'étendant à partir de la première bride s'étendant vers l'extérieur (34) à environ quatre-vingt-dix degrés et se terminant au niveau d'une deuxième bride s'étendant vers l'extérieur (48), s'étendant à partir des quatre parois supérieures (24, 26, 28, 30) à environ quatre-vingt-dix degrés ; et
des intersections et des coins arrondis pour éliminer les coins aigus et faciliter le retrait du bac (20) d'un moule ; et
ledit bac (20) étant composé d'un plastique transparent à transmission de la lumière ultraviolette, capable de résister à l'exposition à des environnements de stérilisation à la vapeur et à l'oxyde d'éthylène sans entraîner une dégradation du bac (20).
